# EUROPÄISCHE PATENTANMELDUNG

(11) **EP 0 639 389 A1**
(43) Veröffentlichungstag der Anmeldung: **22.02.1995**
(21) Anmeldenummer: 94890134.3
(22) Anmeldetag: 12.08.1994
(51) Int. Cl.: A61M 39/26

(54) **Kupplung für die Verbindung von Schlauchleitungen**

(30) Priorität: 17.08.1993 AT 1642/93
(71) Anmelder: Dieringer, Franz A., A-2371 Hinterbrühl (AT)
(72) Erfinder: Dieringer, Franz A., A-2371 Hinterbrühl (AT)
(74) Vertreter: Haffner, Thomas M., Dr.

(57) **Zusammenfassung**

Bei einer Kupplung für die Verbindung von Schlauchleitungen für medizinische Zwecke mit einem ersten Kupplungsteil (1), in welchem ein Durchtrittsöffnungen (4,5) aufweisender hohler Dorn (3) für den Anschluß eines ersten Schlauchstückes gelagert ist, welcher Dorn in einen mit dem ersten Kupplungsteil (1) lösbar verbundenen zweiten Kupplungsteil (15) mündet, wobei der zweite Kupplungsteil (15) wenigstens zwei Anschlußöffnungen (17,18) und ein verschiebbares Verschlußstück aufweist, sind die Anschlußöffnungen (17,18) des zweiten Kupplungsstückes (15) in Achsrichtung versetzt angeordnet, wobei das Verschlußstück (19) wenigstens eine Ausnehmung oder Durchbrechung aufweist, welche in einer ersten Verschiebelage des Verschlußstückes (19) die beiden Anschlußöffnungen (17,18) des zweiten Kupplungsteiles (15) verbindet, und in einer zweiten Verschiebelage voneinander trennt, wobei eine Anschlußöffnung (18) des zweiten Kupplungsteiles (15) in der zweiten Verschiebelage des Verschlußstückes (19) mit dem hohlen Dorn (3) des ersten Kupplungsteiles (1) in offener Verbindung steht.

## Beschreibung

Die Erfindung bezieht sich auf eine Kupplung für die Verbindung von Schlauchleitungen für medizinische Zwecke mit einem ersten Kupplungsteil, in welchem ein Durchtrittsöffnungen aufweisender hohler Dorn für den Anschluß eines ersten Schlauchstückes gelagert ist, welcher in einen mit dem ersten Kupplungsteil lösbar verbundenen zweiten Kupplungsteil mündet, wobei der zweite Kupplungsteil wenigstens zwei Anschlußöffnungen und ein verschiebbares Verschlußstück aufweist.

Eine Kupplung der eingangs genannten Art ist beispielsweise der AT-PS 394 657 bereits zu entnehmen. Eine weitere Einrichtung ähnlicher Art ist aus der WO-A1-90/07953 bekannt geworden. Bei den bekannten sterilen Verbindungen für Schlauchleitungen für medizinische Zwecke ist jeweils ein hohler Dorn axial verschiebbar in einem Kupplungsteil gehalten. Das freie, dem Schlauchanschluß des Dornes abgewandte Ende bewegt sich bei axialer Verschiebung des Dornes durch elastisch verformbare Einlagen, welche mit einem Desinfektionsmittel imprägniert sein können. Die Verschiebung erfolgt, bis die Mündung des axial verschiebbaren Dornes in einen zweiten Kupplungsteil eintaucht, wobei dieser zweite, mit dem ersten Kupplungsteil verbindbare Kupplungsteil wenigstens zwei Öffnungen aufweist. Eine der beiden Öffnungen wird in der Endlage des Dornes durch Verschiebung verschlossen, wobei über die zweite Öffnung des zweiten Kupplungsteiles Infusionsflaschen, Beutel od. dgl. direkt mit einem Patienten verbunden werden können. Zum Wechseln derartiger Infusionsflaschen od. dgl. mußte der axial verschiebbare Dorn wiederum zurückgezogen werden, worauf nach Wechsel der Infusionsflaschen, Beutel od. dgl. der hohle Dorn wiederum in seine Verschiebelage bewegt werden konnte, in welcher zwischen der Infusionsflasche und dem hohlen Dorn wiederum eine offene Verbindung herrscht. Nachteilig bei den bekannten Konstruktionen war hiebei, daß bei jedem Verschieben des Dornes ein mehr oder minder großes Flüssigkeitsvolumen aus dem Kupplungsteil ausgepreßt wurde. Im übrigen war die Handhabung der Kupplung relativ aufwendig, da, bedingt durch die erforderliche Dichtheit, hier mit sehr engen Toleranzen gefertigt werden mußte, wodurch die Verschiebekräfte relativ hoch waren.

Die Erfindung zielt nun darauf ab, eine Kupplung der eingangs genannten Art zu schaffen, bei welcher der Manipulationsaufwand wesentlich herabgesetzt ist, und welche bei geringem Kraftaufwand eine sichere Manipulation und insbesondere einen sicheren Wechsel von Infusionsbestecken ermöglicht. Gleichzeitig zielt die Erfindung darauf ab, die Kupplung so auszubilden, daß bei einer Umschaltung von Anschlußöffnungen im zweiten Kupplungsstück keine Flüssigkeitsvolumina verdrängt werden müssen, und somit ein sauberes und steriles Arbeiten in jeder Phase gewährleistet ist. Zur Lösung dieser Aufgabe besteht die erfindungsgemäße Kupplung im wesentlichen darin, daß die Anschlußöffnungen des zweiten Kupplungsstückes in Achsrichtung versetzt angeordnet sind, und daß das Verschlußstück wenigstens eine Ausnehmung oder Durchbrechung aufweist, welche in einer ersten Verschiebelage des Verschlußstückes die beiden Anschlußöffnungen des zweiten Kupplungsteiles verbindet, und in einer zweiten Verschiebelage voneinander trennt, wobei eine Anschlußöffnung des zweiten Kupplungsteiles in der zweiten Verschiebelage des Verschlußstückes mit dem hohlen Dorn des ersten Kupplungsteiles in offener Verbindung steht. Dadurch, daß die Anschlußöffnugen des zweiten Kupplungsstückes in Achsrichtung versetzt angeordnet sind und das Verschlußstück wenigstens eine Ausnehmung oder Durchbrechung aufweist, welche in einer ersten Verschiebelage des Verschlußstückes die beiden Anschlußöffnungen des zweiten Kupplungsteiles verbindet und in einer zweiten Verschiebelage voneinander trennt, wird eine Verschiebung des Verschlußstückes ermöglicht, bei welcher kein Flüssigkeitsvolumen verdrängt werden muß und in einer ersten Verschiebelage die beiden Anschlußöffnungen miteinander in fluchtender Verbindung stehen, wohingegen in einer zweiten Verschiebelage die Anschlußöffnungen des zweiten Kupplungsstückes sicher voneinander getrennt sind. In dieser zweiten Verschiebelage ist es nun möglich, eine Anschlußöffnung mit dem hohlen Dorn in offene Verbindung zu setzen. Die erfindungsgemäße Konstruktion ist in der Manipulation wesentlich vereinfacht und dadurch, daß das Auspressen von Flüssigkeitsvolumina bei Verschieben des Verschlußstückes vermieden wird, wird das Arbeiten mit der erfindungsgemäßen Kupplung wesentlich erleichtert. Um auch auf eine axiale Verschiebung des Dornes und damit einen erhöhten Manipulationsaufwand verzichten zu können, ist die Ausbildung mit Vorteil so getroffen, daß mit dem Dorn wenigstens eine Ventilhülse oder ein Ventilstempel axial verschieblich verbunden ist, deren Stirnfläche bzw. Sitzfläche federnd gegen einen Ringbord bzw. einen Ventilsitz des Dornes gepreßt ist, wobei die Ventilhülse bei Kupplung mit dem zweiten Kupplungsteil dichtend gegen eine Anlagefläche des zweiten Kupplungsteiles preßbar und in dieser Lage gehalten ist und wobei die Ventilhülse bzw. der Ventilstempel in axialer Richtung unter Freigabe der Durchtrittsöffnungen des Dornes verschiebbar ist. Die axial verschiebliche Ventilhülse bzw. der Ventilstempel wird hiebei beim Festlegen des zweiten Kupplungsstückes zurückgeschoben und gibt die Durchtrittsöffnung des Dornes frei, wobei bei diesem Verschiebeweg der Ventilhülse gleichzeitig die geforderte Sterilität der Verbindung gewährleistet werden kann und eine präzise Abdichtung erfolgen kann. Insgesamt wird eine Ausbildung erzielt, bei welcher in jeder beliebigen Drehlage der Kupplungsstücke eine sichere sterile Verbindung ermöglicht wird.

Mit Vorteil ist die Ausbildung hiebei so getroffen, daß der Durchmesser des Ringbordes im wesentlichen dem Innendurchmesser einer axialen Bohrung des zweiten Kupplungsteiles entspricht, in welche Bohrung in axialer Richtung derselben versetzt die beiden Anschlußöffnungen münden. Auf diese Weise taucht der Ringbord des Dornes weitestgehend dichtend in die Bohrung des zweiten Kupplungsteiles ein, sodaß auch hier die gewünschte Sterilität und eine besonders einfache Bedienung gewährleistet ist.

Das Verschlußstück selbst kann entweder Bohrungen oder außen eine Umfangsnut zur Verbindung der beiden Anschlußöffnungen des zweiten Kupplungsstückes aufweisen. Gemäß einer besonders bevorzugten Ausbildung ist das Verschlußstück als in der Bohrung des zweiten Kupplungsteiles dichtend verschieblicher Kolben ausgebildet, wobei vorzugsweise die Ausnehmung des Verschlußstückes als Umfangsnut ausgebildet ist, deren Breite in Achsrichtung größer ist oder gleich dem Abstand der Anschlußöffnungen des zweiten Kupplungsstückes voneinander ist. Auf diese Weise wird bei der Verschiebung des Verschlußstückes lediglich das in der Ringnut enthaltene Flüssigkeitsvolumen verschoben, ohne daß es zu einer Auspressung von Flüssigkeiten kommt, und es wird eine besonders einfache und betriebssichere Konstruktion gewährleistet.

In besonders vorteilhafter Weise ist die Ausbildung so getroffen, daß die dem Dorn zugewandte Seite des Kolbens eine Zentrieröffnung für einen über den Ringbord des Dornes vorragenden Teil des Dornes trägt. Eine derartige Zentrierung bei der Verschiebung ermöglicht es, den Kraftaufwand beim Anordnen des zweiten Kupplungsteiles und beim Verschieben des Verschlußgliedes weiter zu verringern, wobei mit Vorteil die Ausbildung so getroffen ist, daß die Ventilhülse von zwei axial aneinander anschließenden Teilen gebildet ist, deren am Ringbord in Anlage bringbarer Teil eine konische dichtende Außenfläche aufweist. Auf diese Weise wird ein dichter Anschluß durch die federnd gegen den zweiten Kupplungsteil gepreßte Ventilhülse bzw. durch einen Teil dieser Ventilhülse gewährleistet, wofür mit Vorteil die Ausbildung so getroffen ist, daß der zweite Kupplungsteil unter Kompression einer die Ventilhülse abstützenden Feder mit dem ersten Kupplungsteil verbindbar ist.

Die erfindungsgemäße Konstruktion erlaubt es, auf eine axiale Verschiebung des Dornes relativ zum ersten Kupplungsteil zu verzichten, wodurch, wie bereits eingangs erwähnt, die Handhabung wesentlich vereinfacht wird. Mit Vorteil ist die Ausbildung hiebei so getroffen, daß der Dorn in axialer Richtung unverschiebbar mit dem ersten Kupplungsteil verbunden ist.

Zur Erzielung der geforderten Sterilität der Verbindung können, wie bereits bei bekannten Konstruktionen, mit Desinfektionsmittel getränkte Schwämme Verwendung finden, wobei mit Vorteil die Ausbildung so getroffen ist, daß ein ein Desinfektionsmittel enthaltender Schwamm in einem axial entgegen der Kraft einer weiteren Feder verschiebbaren Korb od. dgl. im ersten Kupplungsteil verschiebbar gelagert ist, und vorzugsweise ein mit Desinfektionsmittel getränkter, ringförmiger Schwamm an der dem ersten Kupplungsteil zugewandten Stirnfläche des zweiten Kupplungsteiles angeordnet ist. Eine derartige Ausbildung führt dazu, daß bei Austausch des zweiten Kupplungsstückes automatisch neuerlich Desinfektionsmittel eingebracht wird, und der Schwamm des ersten Kupplungsstückes neuerlich mit Desinfektionsmittel getränkt wird, sodaß die Sterilität der Verbindung erhalten bleibt.

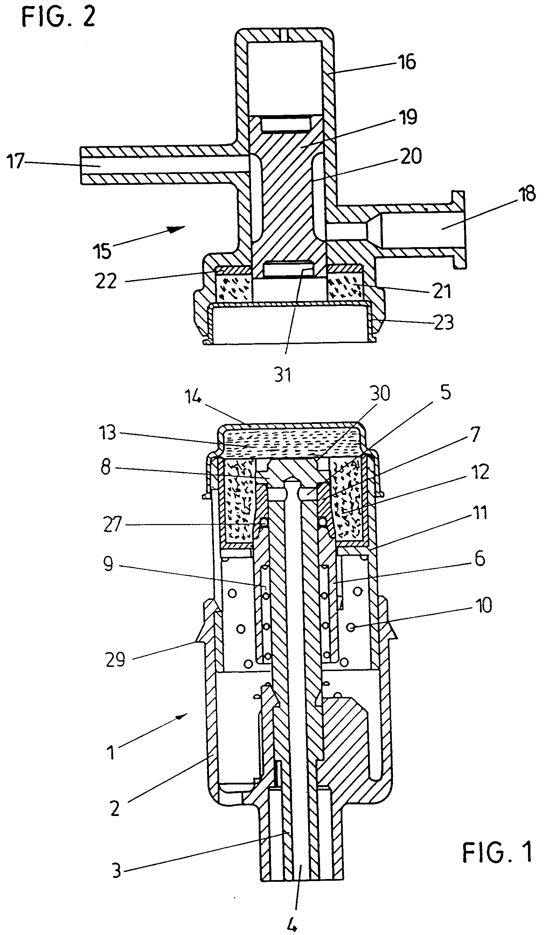

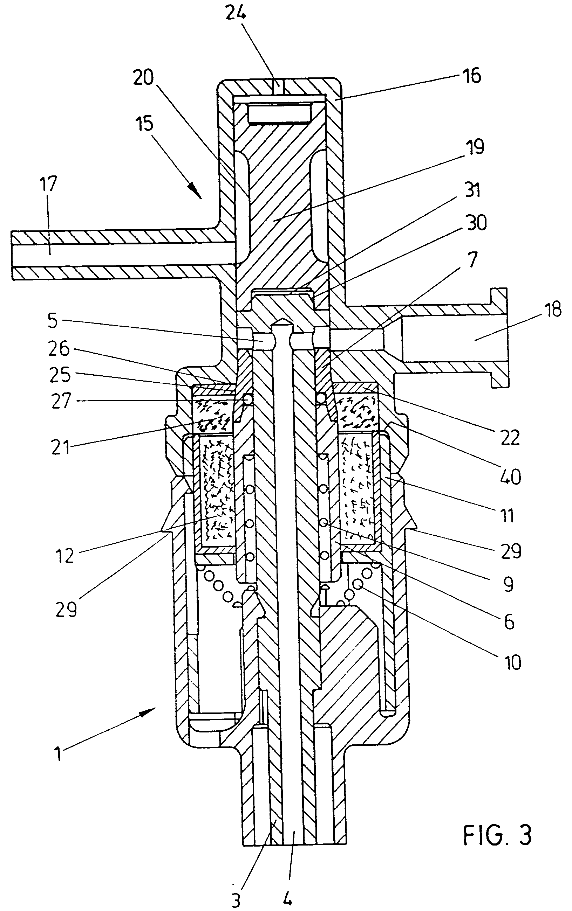

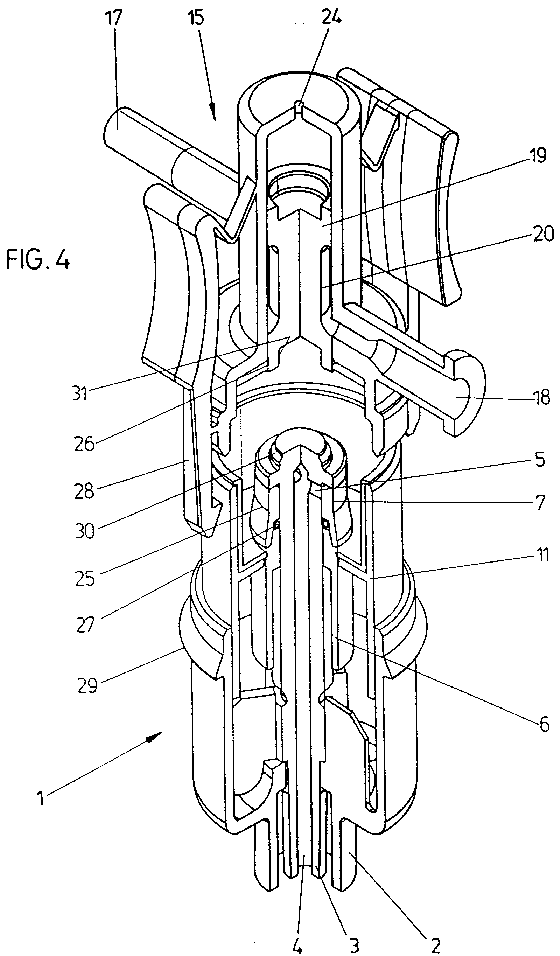

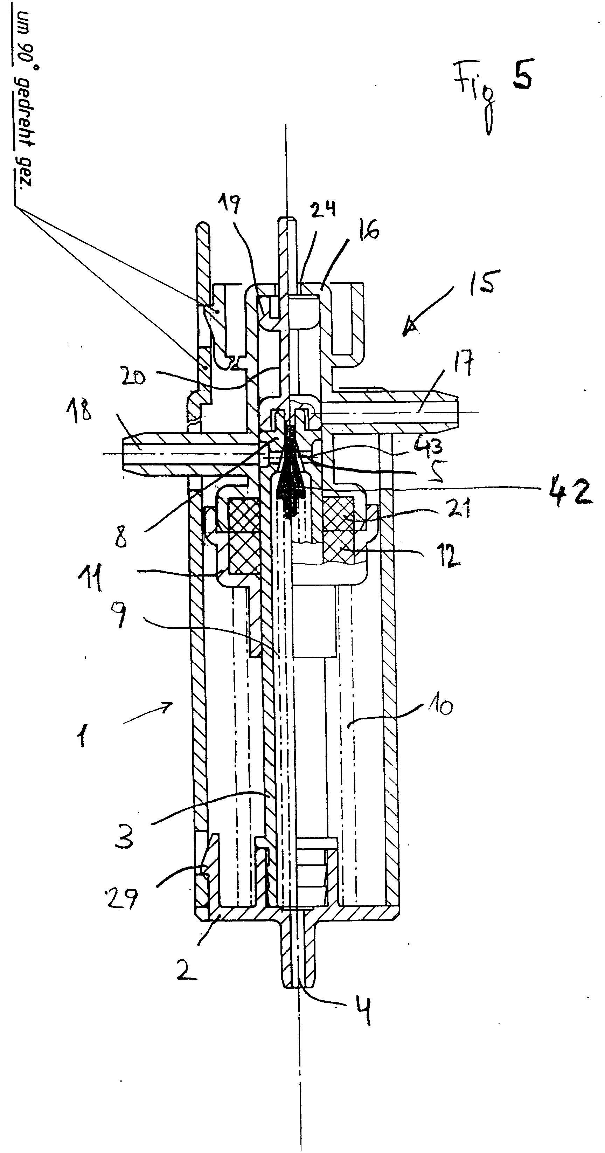

Die Erfindung wird nachfolgend anhand eines in einer Zeichnung schematisch dargestellten Ausführungsbeispieles näher erläutert. In dieser zeigen Fig. 1 einen Schnitt durch einen ersten Kupplungsteil der erfindungsgemäßen Kupplung; Fig. 2 einen Schnitt durch einen zweiten Kupplungsteil der erfindungsgemäßen Kupplung; Fig. 3 einen Schnitt durch die erfindungsgemäße Kupplung, wobei die Kupplungsteile der Fig. 1 und 2 in zusammengebautem Zustand dargestellt sind; Fig. 4 eine perspektivische Schnittdarstellung der erfindugsgemäßen Kupplung in zusammengebautem Zustand und Fig. 5 eine abgewandelte Ausbildung in einer Darstellung entsprechend der Fig. 3.

Der in Fig. 1 dargestellte erste Kupplungsteil 1 weist einen starr mit dem Gehäuse 2 des ersten Kupplungsteiles 1 verbundenen hohlen Dorn 3 auf, an welchen ein nicht näher dargestelltes und mit einem Patienten verbundenes Schlauchstück festlegbar ist. Der hohle Dorn 3 weist einen Duchtrittskanal 4 auf, welcher in Durchtrittsöffnungen 5 mündet, welche in zusammengestecktem Zustand des Kuppungsteiles 1 mit einem zweiten Kupplungsteil 15 mit Anschlußöffnungen dieses zweiten Kupplungsteiles fluchten. Am Außenumfang des hohlen Dornes 3 ist eine Ventilhülse verschieblich gelagert, welche aus zwei axial aneinander anschließenden Teilen 6 und 7 gebildet ist. In dem in Fig. 1 dargestellten abgeschlossenen Zustand des ersten Kupplungsteiles 1 werden die Durchtrittsöffnungen 5 des hohlen Dornes 3 von dem Ventilteil 7 überschliffen und von diesem abgedichtet. Der Ventilteil 7 ist hiebei an einem Ringbord 8 des hohlen Dornes 3 abgestützt und in dieser Lage durch eine Druckfeder 9 gehalten. Die Druckfeder 9 ist hiebei im Ventilteil 6 geführt.

Eine Druckfeder 10 beaufschlagt einen im Gehäuse 2 des ersten Kupplungsteiles 1 verschiebbaren Korb 11, in welchem ein mit Desinfektionsmittel getränkter und den Ventilteil 7 umgebender Schwamm 12 angeordnet ist. In dem in Fig. 1 dargestellten abgeschlossenen Zustand des ersten Kupplungsteiles 1 ist dieser durch einen weiteren Schwamm 13 und eine Abdeckkappe 14 steril abgeschlossen. Ein Dichtring 27 dichtet die Ventilhülse gegen den hohlen Dorn 3 ab.

Der in Fig. 2 dargestellte zweite Kupplungsteil 15 weist ein Gehäuse 16 auf, an welchem Anschlußöffnungen 17 und 18 in axialer Richtung dieses zweiten Kupplungsteiles 15 versetzt angeordnet sind. Im zweiten Kupplungsteil 15 ist als Verschlußstück 19 ein Kolben dichtend verschieblich geführt, wobei der Kolben 19 an seinem Außenumfang eine Ausnehmung bzw. Umfangsnut 20 aufweist, welche in dem in Fig. 2 dargestellten unbetätigten Zustand des zweiten Kupplungsteiles 15 eine Verbindung zwischen den Anschlußöffnugen 17 und 18 freigibt. In diesem Zustand kann der zweite Kupplungsteil 15 entlüftet werden oder es kann über die Nadel- oder Entlüftungsbohrung 17 eine Flüssigkeit angesaugt werden. Im zweiten Kupplungsteil 15 ist ebenfalls ein mit Desinfektionsmittel getränkter Schwamm 21 sowie eine Abstreiflippe 22 angeordnet, wobei diese Elemente entsprechend dem Außendurchmesser des im gekoppelten Zustand eintretenden hohlen Dornes ringförmig ausgebildet sind. Auch der zweite Kupplungsteil 15 ist vor seinem Gebrauch durch eine Abdeckkappe 23 steril abgedichtet.

In dem in Fig. 3 dargestellten gekoppelten Zustand der beiden Kupplungsteile 1 und 15 ist ersichtlich, daß die Durchtrittsöffnungen 5 des hohlen Dornes 3 direkt in die Spritzen- oder Infusionsöffnung 18 des zweiten Kupplungsteiles 15 münden. Durch die Kopplung der beiden Kupplungsteile 1 und 15 wurde das Verschlußstück 19 in eine Position bewegt, in welcher keine Verbindung mehr zwischen den Anschlußöffnungen 17 und 18 des zweite Kupplungsteiles 15 über die Umfangsnut 20 des Kolbens 19 freigegeben wird. Für die Verschiebebewegung des Kolbens 19 ist, an der Stirnseite des zweiten Kupplungsteiles 15 eine Entlastungsbohrung 24 vorgesehen. Für eine Zentrierung des Dorns 3 bei der Kopplung weist dieser an seiner Stirnseite einen abgesetzten Bereich 30 auf, welcher in eine entsprechende Ausnehmung 31 am Kolben 19 einpaßbar ist.

Die in ungebrauchtem Zustand durch den Ventilteil 7 abgeschlossenen Durchtrittsöffnugen 5 werden bei der Kupplung der Kupplungsteile dadurch freigegeben, daß eine Anlagefläche 25 des Ventilteiles 7 in Anlage an eine Schulter 26 des zweiten Kupplungsteiles 15 gelangt, wodurch bei weiterer Einschiebebewegung des ersten Kupplungsteiles 1 in den zweiten Kupplungsteil 15 eine Verschiebung der zweiteiligen Ventilhülse 6, 7 erfolgt. Bereits zuvor wird der Korb 11 gegen eine Fläche 40 gepreßt und entgegen der Kraft der Feder 10 zurückgeschoben. Für eine vollständige Abdichtung ist zwischen den Ventilteilen 6 und 7 und dem hohlen Dorn 3 der Dichtring 27 vorgesehen.

Dadurch, daß als Verschlußstück ein mit einer Umfangsnut ausgebildeter Kolben 19 verwendet wird, wird bei Verschiebung des Kolbens beim Zusammenstecken der Kupplung das in der Ausnehmung 20 enthaltene Flüssigkeitsvolumen mitgenommen und es tritt keine Flüssigkeit bei der Nadelbohrung 17 aus. An Stelle der Umfangsnut 20 kann auch eine Durchtrittsbohrung im Kolben 19 vorgesehen sein, deren Enden auf den axialen Abstand der Anschlußöffnungen 17 und 18 des zweiten Kupplungsstückes 15 abgestimmt sein müssen, wobei der Kolben 19 verdrehgesichert werden muß.

Zum Lösen der in Fig. 3 dargestellten Verbindung zwischen einem Patienten und einer an der Anschlußöffnung 18 angeschlosssenen Spritze oder Infusion wird durch eine Betätigung von am zweiten Kupplungsteil 15 ausgebildeten Verriegelungshaken 28, welche in Fig. 4 im Detail dargestellt sind und mit dem Verrastungsbund 29 am Außenumfang des ersten Kupplungsteiles zusammenwirken, die Verbindung gelöst. Bei Abnehmen des zweiten Kupplungsteiles 15 werden die Ventilteile 6 und 7 durch Beaufschlagung mittels der Kraft der Federn 9 und 10 axial am Außenumfang des hohlen Dornes 3 verschoben, wodurch die Durchtrittsöffnungen 5 abgeschlossen werden. Durch die ebenfalls axiale Verschiebung des Korbes 11 und des darin enthaltenen Schwammes 12 befinden sich der Kopf des hohlen Dornes 3 sowie die Ventilteile 6 und 7 wieder in einer desinfiszierenden Umgebung. Der zweite Kupplungsteil 15 kann somit abgezogen werden und als Einwegteil entsorgt werden, worauf mit dem ersten Kupplungsteil entweder ein weiterer zweiter Kupplungsteil verbunden wird oder der erste Kupplungsteil durch eine neue einen Schwamm 13 enthaltende Abdeckkappe 14 wiederum steril verschlossen wird.

In der perspektivischen Darstellung gemäß Fig. 4 sind die wesentlichen Bestandteile der beiden Kupplungsteile 1 und 15 in ihrer Position unmittelbar vor der Kupplung dargestellt, wobei der Übersichtlichkeit halber beispielsweise die Federn und Schwammelemente nicht eingezeichnet sind.

Bei der abgewandelten Ausbildung nach Fig. 5 wird anstelle der Ventilhülse 6,7 ein Ventilstempel 42 mit konischen Sitzflächen verwendet. Die Öffnung des Ventiles durch Abheben von der Sitzfläche 43 des Ventiles wird durch einen Vorsprung 41 des Schließgliedes 19 bewirkt. Die übrigen mit den gleichen Bezugszeichen, wie zuvor bezeichneten Bauteile, entsprechen funktionell den entsprechenden Teilen in der Darstellung nach Fig. 3.

## Patentansprüche

1. Kupplung für die Verbindung von Schlauchleitungen für medizinische Zwecke mit einem ersten Kupplungsteil (1), in welchem ein Durchtrittsöffnungen (5) aufweisender hohler Dorn (3) für den Anschluß eines ersten Schlauchstückes gelagert ist, welcher Dorn (3) in einen mit dem ersten Kupplungsteil (1) lösbar verbundenen zweiten Kupplungsteil (15) mündet, wobei der zweite Kupplungsteil (15) wenigstens zwei Anschlußöffnungen (17,18) und ein verschiebbares Verschlußstück (19) aufweist, dadurch gekennzeichnet, daß die Anschlußöffnungen (17,18) des zweiten Kupplungsstückes (15) in Achsrichtung versetzt angeordnet sind, und daß das Verschlußstück (19) wenigstens eine Ausnehmung oder Durchbrechung (20) aufweist, welche in einer ersten Verschiebelage des Verschlußstückes (19) die beiden Anschlußöffnungen (17,18) des zweiten Kupplungsteiles (15) verbindet, und in einer zweiten Verschiebelage voneinander trennt, wobei eine Anschlußöffnung des zweiten Kupplungsteiles (15) in der zweiten Verschiebelage des Verschlußstückes (19) mit dem hohlen Dorn (3) des ersten Kupplungsteiles (1) in offener Verbindung steht.

2. Kupplung nach Anspruch 1, dadurch gekennzeichnet, daß mit dem Dorn (3) wenigstens eine Ventilhülse (6,7) oder ein Ventilstempel (42) axial verschieblich verbunden ist, deren Stirnfläche bzw. Sitzfläche federnd gegen einen Ringbord (8) bzw. einen Ventilsitz (43) des Dornes (3) gepreßt ist, wobei die Ventilhülse (6,7) bei Kupplung mit dem zweiten Kupplungsteil (15) dichtend gegen eine Anlagefläche (25) des zweiten Kupplungsteiles (15) preßbar und in dieser Lage gehalten ist und wobei die Ventilhülse (6,7) bzw. der Ventilstempel (42) in axialer Richtung unter Freigabe der Durchtrittsöffnungen (5) des Dornes (3) verschiebbar ist.

3. Kupplung nach 2, dadurch gekennzeichnet, daß der Durchmesser des Ringbordes (8) im wesentlichen dem Innendurchmesser einer axialen Bohrung des zweiten Kupplungsteiles (15) entspricht, in welche Bohrung in axialer Richtung derselben versetzt die beiden Anschlußöffnungen (17,18) münden.

4. Kupplung nach Anspruch 1, 2 und 3, dadurch gekennzeichnet, daß das Verschlußstück (19) als in der Bohrung des zweiten Kupplungsteiles (15) dichtend verschieblicher Kolben ausgebildet ist.

5. Kupplung nach einem der Ansprüche 1 bis 4, dadurch gekennzeichnet, daß die Ausnehmung (20) des Verschlußstückes (19) als Umfangsnut ausgebildet ist, deren Breite in Achsrichtung größer ist oder gleich dem Abstand der Anschlußöffnungen (17,18) des zweiten Kupplungsstückes (15) voneinander ist.

6. Kupplung nach einem der Ansprüche 1 bis 5, dadurch gekennzeichnet, daß die dem Dorn (3) zugewandte Seite des Kolbens (19) eine Zentrieröffnung für einen über den Ringbord (8) des Dornes (3) vorragenden Teil des Dornes (3) trägt.

7. Kupplung nach einem der Ansprüche 2 bis 6, dadurch gekennzeichnet, daß die Ventilhülse (6,7) von zwei axial aneinander anschließenden Teilen gebildet ist, deren am Ringbord (8) in Anlage bringbarer Teil eine konische dichtende Außenfläche aufweist, wobei zwischen den axial aneinander anschließenden Teilen ein Dichtring (27) enthalten ist.

8. Kupplung nach einem der Ansprüche 2 bis 7, dadurch gekennzeichnet, daß der zweite Kupplungsteil (15) unter Kompression einer die Ventilhülse (6,7) abstützenden Feder (9) mit dem ersten Kupplungsteil (1) verbindbar ist.

9. Kupplung nach einem der Ansprüche 1 bis 8, dadurch gekennzeichnet, daß der Dorn (3) in axialer Richtung unverschiebbar mit dem ersten Kupplungsteil (1) verbunden ist.

10. Kupplung nach einem der Ansprüche 1 bis 9, dadurch gekennzeichnet, daß ein ein Desinfektionsmittel enthaltender Schwamm (12) in einem axial entgegen der Kraft einer weiteren Feder (10) verschiebbaren Korb (11) od. dgl. im ersten Kupplungsteil (1) verschiebbar gelagert ist.

11. Kupplung nach einem der Ansprüche 1 bis 10, dadurch gekennzeichnet, daß ein mit Desinfektionsmittel getränkter, ringförmiger Schwamm (21) an der dem ersten Kupplungsteil (1) zugewandten Stirnfläche des zweiten Kupplungsteiles (15) angeordnet ist.
